# EUROPEAN PATENT SPECIFICATION

(11) **EP 3 980 086 B1**
(45) Date of publication and mention of the grant of the patent: **18.03.2026**
(21) Application number: 20750430.9
(22) Date of filing: 05.06.2020
(51) Int. Cl.: A61L 27/34, A61L 27/36, A61L 31/10

(54) **COATED POLYMERIC MATERIAL**
BESCHICHTETES POLYMERMATERIAL
MATÉRIAU POLYMÈRE REVÊTU

(30) Priority: 07.06.2019 US 201962858740 P
(43) Date of publication of application: 13.04.2022
(73) Proprietor: LifeCell Corporation, North Chicago, IL 60064 (US)
(72) Inventor: XU, Hui, Plainsboro, NJ 08536 (US); HUANG, Li, Ting, Branchburg, NJ 08853 (US); STEC, Eric, Washington, NJ 07882 (US); POMERLEAU, Ming, F., Califon, NJ 07830 (US)
(74) Representative: Hoffmann Eitle
(86) International application number: PCT/US2020/036427
(87) International publication number: WO 2020/247822

(56) References cited:
- US-A1- 2018 214 520

## Description

This application claims priority under 35 U.S.C. § 119 to U.S. Provisional Application No. 62/854,740, filed June 7, 2019.

The present disclosure relates to tissue products, including polymeric materials that are treated with or coated by a coating of acellular tissue matrix particles, transglutaminase, and an at least partially denatured collagen.

Various tissue-derived products are used to regenerate, repair, or otherwise treat diseased or damaged tissues and organs. Such products can include intact tissue grafts or acellular or reconstituted acellular tissues (e.g., acellular tissue matrices from skin, intestine, or other tissues, with or without cell seeding). Such products can also include hybrid or composite materials, e.g., materials including a synthetic component such as a polymeric mesh substrate with a coating or covering that includes materials derived from tissue. US patent application publication no. 2018/0214520 discloses the use of transglutaminases to treat various tissue matrix products.

US2018/214602 A1 discloses a medical device, comprising a collagen-containing tissue matrix; and a transglutaminase coating disposed on an outer surface of the implant, wherein the coating provides at least one of an anti-adhesion or anti-abrasion property to the outer surface. The collagen-containing tissue matrix can comprise a particulate acellular tissue matrix. The devices can provide one or more of improved resistance to surface damage, improved resistance to wear, resistance to formation of adhesions with surrounding tissues, or reduced friction when in contact with other materials.

Accordingly, the present application provides devices and methods that provide modified tissue products with transglutaminase coatings. The devices and methods can provide one or more of improved resistance to surface damage, improved resistance to wear, resistance to formation of adhesions with surrounding tissues, or reduced friction when in contact with other materials.

### SUMMARY

The present invention relates to a tissue composition as defined in claim 1. The tissue composition includes a polymeric material, and a coating disposed on at least a surface of the polymeric material. The coating includes a group of acellular tissue matrix particles, transglutaminase, and an at least partially denatured collagen. In some embodiments, the group of acellular tissue matrix particles comprise acellular dermal tissue matrix particles. In some embodiments, the group of acellular tissue matrix particles comprise porcine acellular tissue matrix particles. In some embodiments, the group of acellular tissue matrix particles are treated with an enzymatic solution. In further embodiments, the enzymatic solution comprises a proteolytic enzyme. In some embodiments, the composition is freeze-dried. In some embodiments, the coating comprises about 0.1% to 25% of the acellular tissue matrix particles. In some embodiments, the coating comprises about 0.5% to 10% of the transglutaminase. In some embodiments, the coating comprises about 0.5% to 10% of the gelatin. In some embodiments, the polymeric material is a synthetic polymer. In some embodiments, the polymeric material is biodegradeable. In some embodiments, the polymeric material is polypropylene. In some embodiments, the at least partially denatured collagen is a gelatin. In further embodiments, the gelatin is a transglutaminase treated gelatin.

Also disclosed herein, but not claimed, is a method of producing a tissue composition is provided. The method can include suspending a group of acellular tissue matrix particles in a solution, mixing the solution with transglutaminase, mixing the solution with an at least partially denatured collagen, and coating a polymeric material with the solution. In some embodiments, the group of acellular tissue matrix particles comprise acellular dermal tissue matrix particles. In some embodiments, the group of acellular tissue matrix particles comprise porcine acellular tissue matrix particles.

In some embodiments, the method further includes treating the group of acellular tissue matrix particles with an enzymatic solution. In further embodiments, the enzymatic solution comprises a proteolytic enzyme.

In some embodiments, the solution comprises about 0.1% to 25% of the acellular tissue matrix particles. In some embodiments, the solution comprises about 0.5% to 10% of the transglutaminase. In some embodiments, the solution comprises about 0.5% to 10% of the gelatin.

In some embodiments, coating the polymeric material includes pouring a portion of the solution into a mold, placing the polymeric material on top of the solution, and pouring the remaining solution over the polymeric material. In some embodiments, the method further includes freeze-drying the coated polymeric material. In some embodiments, the method further includes stabilizing the coated polymeric material with dehydrothermal treatment. In some embodiments, the polymeric material is a synthetic polymer. In some embodiments, the polymeric material is biodegradeable. In some embodiments, the polymeric material is polypropylene. In some embodiments, the at least partially denatured collagen is a gelatin. In further embodiments, the gelatin is a transglutaminase treated gelatin.

### DESCRIPTION OF THE DRAWINGS

Fig. 1 is a flowchart depicting a method of producing a coated polymeric material according to an embodiment.
Fig. 2 depicts a top view and cross-sectional view of a coated polymeric material according to an embodiment.
Fig. 3 is a bar graph depicting the maximum tensile strengths exhibited by an exemplary coated polymeric material.
Fig. 4 depicts images of tensile load testing of an exemplary coated polymeric material.
Fig. 5 is a bar graph depicting the burst strength of an exemplary coated polymeric material.
Fig. 6 depicts images of burst strength testing of an exemplary coated polymeric material.
Fig. 7 provides scanning electron microscopy (SEM) images of exemplary coated polymeric materials.
Fig. 8 includes hematoxylin & eosin stained sections of polypropylene materials versus an exemplary coated polymeric material after implantation in a rat.
Fig. 9 includes hematoxylin & eosin stained sections of an exemplary coated polymeric material after implantation in a rat.
Fig. 10 are images of gross explants of implants made of polypropylene material versus an exemplary coated polymeric material after 4 weeks implantation in a rat abdominal wall full thickness defect model.
Fig. 11 includes hematoxylin & eosin stained sections of a polypropylene material versus an exemplary coated polymeric material after 4 weeks implantation in a rat abdominal wall full thickness defect model.
Fig. 12 depicts immunofluorescence stained sections using antibodies against specific macrophage phenotypic markers on a polypropylene material versus an exemplary coated polymeric material after 4 weeks implantation in a rat abdominal wall full thickness defect model.
Fig. 13 provides SEM images of exemplary coated polymeric materials at different coating thicknesses.

### DESCRIPTION OF CERTAIN EXEMPLARY EMBODIMENTS

Reference will now be made in detail to certain exemplary embodiments according to the present disclosure, certain examples of which are illustrated in the accompanying drawings. Wherever possible, the same reference numbers will be used throughout the drawings to refer to the same or like parts.

In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Any range described herein will be understood to include the endpoints and all values between the endpoints.

The section headings used herein are for organizational purposes only and are not to be construed as limiting the subject matter described.

Various human and animal tissues can be used to produce products for treating patients. For example, various tissue products for regeneration, repair, augmentation, reinforcement, and/or treatment of human tissues that have been damaged or lost due to various diseases and/or structural damage (e.g., from trauma, surgery, atrophy, and/or long-term wear and degeneration) have been produced. Such products can include, for example, acellular tissue matrices, tissue allografts or xenografts, and/or reconstituted tissues (i.e., at least partially decellularized tissues that have been seeded with cells to produce viable materials).

A variety of tissue products have been produced for treating soft and hard tissues. For example, ALLODERM^{®} and STRATTICE^{®} (LIFECELL CORPORATION, Branchburg, NJ) are two dermal acellular tissue matrices made from human and porcine dermis, respectively. Although such materials are very useful for treating certain types of conditions, it may be desirable to modify the tissue matrices or other tissue products to alter the surface mechanical properties, to improve resistance to wear or damage, to prevent development of adhesions with surrounding tissues, or to reduce friction when the tissue products are in contact with other materials such as body tissue.

Source tissues are used to create acellular tissue matrices used to form various moldable tissue matrix products and compositions. The acellular tissue matrix may originate from a human or an animal tissue matrix. Suitable tissue sources for an acellular tissue matrix may include allograft, autograft, or xenograft tissues. Human tissue may be obtained from cadavers. Additionally, human tissue may be obtained from live donors; i.e. autologous tissue.

The tissue product can include a tissue matrix, such as a decellularized or partially decellularized tissue matrix. Examples of tissues that may be used can include, but are not limited to, skin, parts of skin (e.g., dermis), fascia, muscle (striated, smooth, or cardiac), adipose tissue, pericardial tissue, dura, umbilical cord tissue, placental tissue, cardiac valve tissue, ligament tissue, tendon tissue, blood vessel tissue (such as arterial and venous tissue), cartilage, bone, neural connective tissue, urinary bladder tissue, ureter tissue, and intestinal tissue. For example, a number of biological scaffold materials that may be used for the tissue matrix are described by Badylak et al., "Extracellular Matrix as a Biological Scaffold Material: Structure and Function," Acta Biomaterialia (2008), doi:10.1016/j.actbio.2008.09.013.

Some examples of non-human tissue sources which may be used for xenograft tissue matrices include pig, cow, dog, cat, or other animals from domestic or wild sources and/or any other suitable mammalian or non-mammalian xenograft tissue source. In some exemplary embodiments, the acellular tissue matrix may originate from a source dermal matrix taken from an animal, such as a pig. In one exemplary embodiment, the source dermal matrix may comprise one or more layers of skin that have been removed from an animal.

If porcine or other animal sources are used, the tissue may be further treated to remove antigenic components, such as 1,3-alpha-galactose moieties, which are present in pigs and other mammals, but not humans or certain other primates. In some embodiments, the tissue is obtained from animals that have been genetically modified to lack expression of antigenic moieties, such as 1,3-alpha-galactose, for example. See Xu, Hui, et al., "A Porcine-Derived Acellular Dermal Scaffold that Supports Soft Tissue Regeneration: Removal of Terminal Galactose-α-(1,3)-Galactose and Retention of Matrix Structure," Tissue Engineering, Vol. 15, 1-13 (2009).

Acellular tissue matrices can provide a suitable tissue scaffold to allow cell ingrowth and tissue regeneration. Starting materials for forming an injectable tissue product include an acellular dermal matrix ("ADM"), in some embodiments. In some embodiments, the ADM is a porcine acellular dermal matrix ("pADM"). In some embodiments, the ADM is a human ADM. Other sources of ADM could be used, as previously mentioned. The starting ADM material may comprise substantially non-cross-linked collagen to allow infiltration with host cells, including fibroblasts and vascular elements. Regardless, some degree of collagen cross-linking may result from processing the ADM.

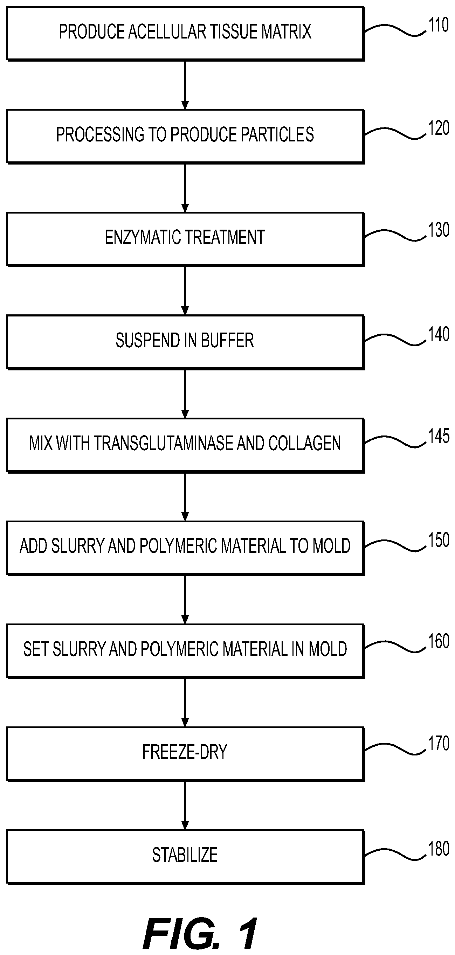

Fig. 1 depicts a flowchart of an exemplary method of producing a coated polymeric material. The method begins at step 110, processing source tissue to produce an acellular tissue matrix. The source tissue may be processed as described above. In some embodiments, the source tissue is dermal tissue. In further embodiments, the tissue is porcine dermal tissue.

Next in Step 120, the acellular tissue matrix is formed into particles. The acellular tissue matrix particles are formed by subjecting the source tissue matrix to mechanical and/or chemical processing steps. Mechanical processing generally removes undesired tissues and reduces the source tissue into smaller particles. For example, a sheet of acellular tissue matrix may be shredded into particles. Mechanical processing may further include grinding, grating, freeze-drying, fracturing, or other processes to break apart tissue. In some embodiments, the acellular tissue matrix is grinded in a meat chopper. The source tissue matrix may be checked for fatty tissue and cut to remove the tissue and/or to prevent tangling of tissue matrix pieces. The source tissue matrix may be frozen and thawed prior to mechanical processing.

In some embodiments, the tissue matrix particles are sorted by size. In an exemplary embodiments, sequentially sized wire screens filter the particles into groups of particles within a similar size range.

Next in Step 130, the acellular tissue matrix particles are treated with an enzyme. Enzymes such as lipases, DNAses, RNAses, alpha-galactosidase, or proteolytic enzymes such as alcalase, tripsin, bromelain, papain, ficin, or other enzymes, may be used to ensure destruction of nuclear materials, antigens from xenogenic sources, residual cellular components, and/or viruses.

Various enzyme activities and treatment times may be used. For example, an enzyme may be provided in a solution with an activity of 1x10⁻⁶ Anson units per mL to 0.015 Anson units per mL, an activity of 1x10⁻⁶ units to 1.5x10⁻³ Anson units per mL, or an activity of about 2x10⁻⁵ Anson units per mL to about 4x10⁻⁵ Anson units per mL. In addition, treatment times may vary between about 4 hours and 5 days.

Step 130 may further include a decellularization treatment. Any conventional method of decellularization may be employed. In some embodiments, multiple decellularization solutions are employed. In further embodiments, a centrifugation and pellet resuspension step follows each treatment with a decellularization solution.

Next in Step 140, the enzyme-treated particles are suspended in a buffer solution. In some embodiments, the buffer includes phosphate buffered saline. In some embodiments, the buffer includes sodium citrate. In further embodiments, the buffer is a 10mM solution of sodium citrate. In a further embodiment, the sodium citrate solution includes 10% solids.

Next in Step 145, the suspended particles are mixed with transglutaminase and at least a partially denatured collagen. The mixture of acellular tissue matrix particles, transglutaminase, and collagen may be a slurry. In some embodiments, the slurry includes a concentration of about 0.1% to 25% acellular tissue matrix particles, about 0.5% to 10% denatured collagen, and about 0.5% to 10% transglutaminase. In further embodiments, the slurry includes a concentration of about 2.5% to 5% acellular tissue matrix particles, about 1.5% to 3% denatured collagen, and about 0.5% to 1% transglutaminase.

Transglutaminases are enzymes expressed in bacteria, plants, and animals that catalyze the binding of gamma-carboxyamide groups of glutamine residues with amino groups of lysine residues or other primary amino groups. Transglutaminases are used in the food industry for binding and improving the physical properties of protein rich foods such as meat, yogurt, and tofu. Transglutaminases are also currently being explored for use in the medical device industry as hydrogels and sealants. See Aberle, T. et al., "Cell-type Specific Four Component Hydrogel," PLoS ONE 9(1): e86740 (Jan. 2004).

For example, the transglutaminase can be provided in a solution or formed into a solution from a stored form (e.g., a dry powder or other suitable storage form). The solution can include any suitable buffer such as phosphate buffered saline or other biologically compatible buffer material that will maintain or support enzymatic activity and will not damage the enzyme or tissue product.

A variety of transglutaminases can be used including any that are biologically compatible, can be implanted in a patient, and have sufficient activity to provide desired catalytic results within a desired time frame. Transglutaminases are known and can include microbial, plant, animal, or recombinantly produced enzymes. Depending on the specific enzyme used, modifications such as addition of cofactors, control of pH, or control of temperature or other environmental conditions may be needed to allow appropriate enzymatic activity. Microbial transglutaminases can be effective because they may not require the presence of metal ions, but any suitable transglutaminase may be used.

In some embodiments, the at least partially denatured collagen is a gelatin. In some embodiments, the gelatin is a porcine gelatin. In further embodiments, the porcine gelatin possess a gel strength (bloom number) of 300. In some embodiments, the gelatin is derived from cold water fish.

Next in Step 150, a portion of the slurry is poured into the bottom of a mold. A "mold" relates to any three-dimensional structure possessing an open area configured to receive the slurry.

A polymeric material is placed within the mold on top of the slurry. The polymeric material can include, for example, a mesh formed of filaments, such as polypropylene. In one aspect, the polymeric material can be substantially non-absorbable or non-biodegradable. In another aspect, the polymeric material can be absorbable. The absorbable mesh can be a polymer selected from the group consisting of polyhydroxyalkanoate, polyglycolic acid, poly-I-lactic acid, polylactic/polyglycolic acid (PLGA), polygalactin 910, and carboxymethyl cellulose. The polymer can include poly-4-hydroxybutyrate. The polymeric material can be a synthetic substrate; the synthetic substrate can include polypropylene. After placement of the polymeric material, the remaining slurry is poured over the polymeric material and previously poured slurry. The coating thickness of the resulting coated material can be controlled by adjusting the amount of slurry poured over the polymeric material.

Next in Step 160, the slurry and the polymeric material are left to set. In some embodiments, the slurry and polymeric material sets overnight. In some embodiments, the slurry and polymeric material sets at room temperature. While the slurry and polymeric material sets, the transglutaminase may cause cross-linking to occur. In some embodiments, the slurry and polymeric material are stored in an environment with a temperature ranging from 0°C to 60°C.

Next in Step 170, the slurry and the polymeric material is freeze-dried to form a coated polymeric material. Freeze-drying produces a tissue product that is not fragile and capable of being stretched. Further, freeze-drying increases the porosity of the tissue product.

Finally in step 180, the coated polymeric material is stabilized with dehydrothermal treatment, such as by heating the material in a vacuum. Dehydrothermal treatment is performed, in one exemplary embodiment, by heating the molded acellular tissue matrix in a vacuum to between about 70°C to about 120°C or between about 80°C and about 110°C or to about 80°C, or any values between the specified ranges in a reduced pressure or vacuum. As used herein, "reduced pressure" means a pressure at least about ten percent (10%) less than the standard atmospheric pressure of 760 mmHg.

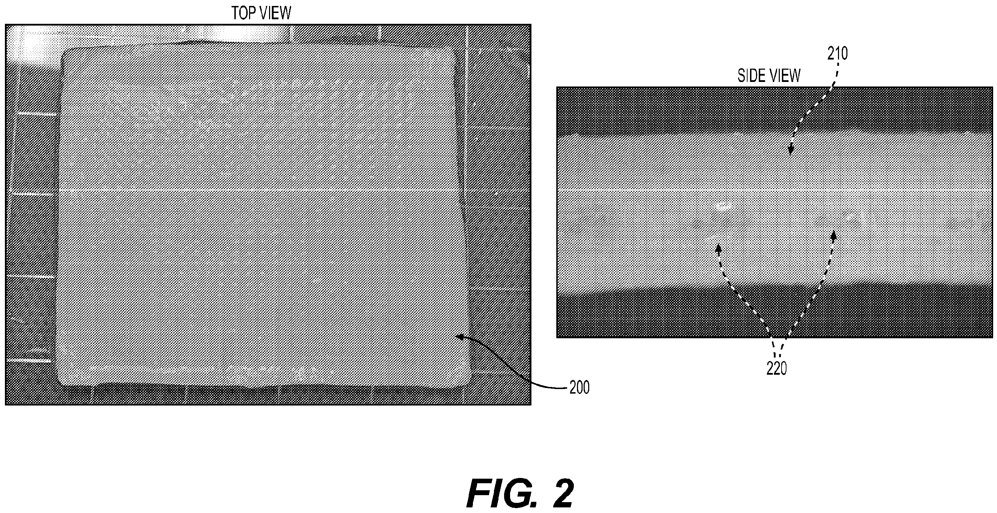

Fig. 2 depicts a top view and cross-sectional view of an exemplary coated polymeric material 200. In some embodiments, coating 210 includes a dried, stabilized mixture of acellular tissue matrix particles, transglutaminase, and at least partially denatured collagen. In some embodiments, the acellular tissue matrix particles are dermal particles. In some embodiments, the acellular tissue matrix particles are porcine particles. In some embodiments, the at least partially denatured collagen is a gelatin. In some embodiments, the coating 210 possesses a three-dimensional structure.

The polymeric material 220 can include, for example, a mesh formed of filaments, such as polypropylene. In one aspect, the polymeric material 220 can be substantially non-absorbable or non-biodegradable. In another aspect, the polymeric material 220 can be absorbable. The absorbable mesh can be a polymer selected from the group consisting of polyhydroxyalkanoate, polyglycolic acid, poly-l-lactic acid, polylactic/polyglycolic acid (PLGA), polygalactin 910, and carboxymethyl cellulose. The polymer can include poly-4-hydroxybutyrate. The polymeric material 220 can be a synthetic substrate; the synthetic substrate can include polypropylene.

The coated polymeric material 200 may be in any form suitable for treatment of a tissue site. In some embodiments, the polymeric material may be in the form of a sheet. Other forms may be produced depending upon the specific polymeric material and intended use of the final tissue product.

The tissue products and their methods of production can be used for the treatment of a variety of conditions. For example, the tissue products may be used to treat hernias (for example, ventral and inguinal hernias), reinforce tendons or ligaments, or in reconstructive surgeries. The tissue products may be used in any application suitable for application of a synthetic or coated synthetic mesh.

### EXAMPLE 1

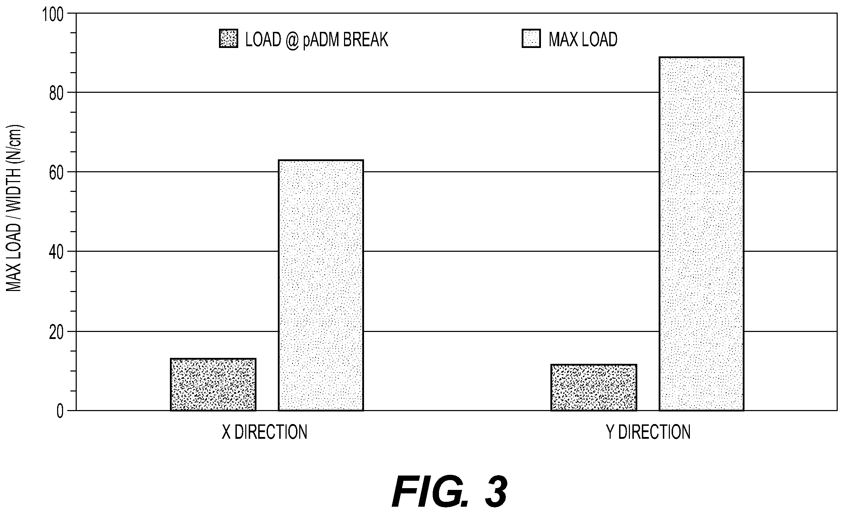

An exemplary coated polymeric material as described above was tested to determine the structural characteristics of the material. The tested coated polymeric material included a concentration of 2.5% acellular tissue matrix, 1.5% denatured collagen, and 0.5% transglutaminase. Fig. 3 depicts the maximum tensile strengths exhibited by an exemplary coated polymeric material. The blue bars of the graph depict the load at which the coating cracks and exposes the polypropylene material. The orange bars depict the load at which the coated material completely breaks.

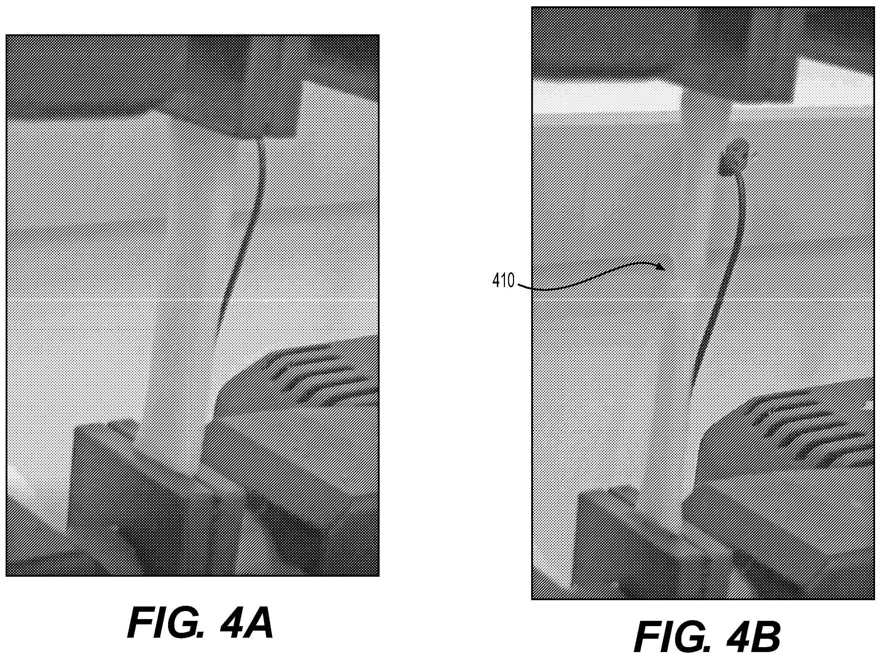

Fig. 4 depicts images of tensile load testing of the exemplary coated polymeric material. Panel A shows the coated material at the start of the test. Panel B shows the coated material at the point when the coating breaks. The breakage occurred at the area labeled 410.

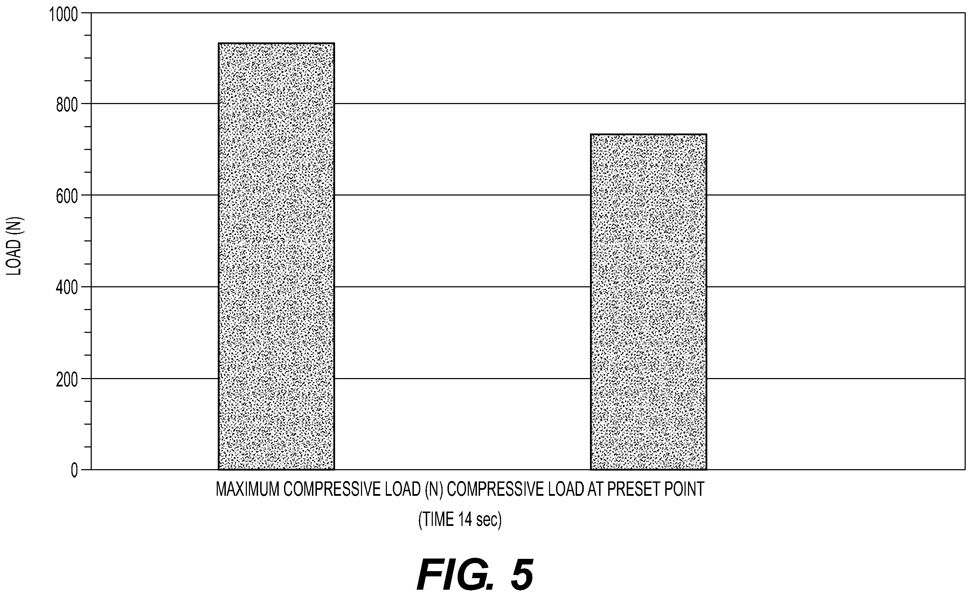

Fig. 5 is a bar graph depicting the burst strength of the exemplary coated polymeric material. Specifically, the graph shows the maximum compression load of the coated material. Maximum compression load refers to the load at which the coated material breaks completely. CompressionLoad at Preset Point refers to the load at which the coating cracks.

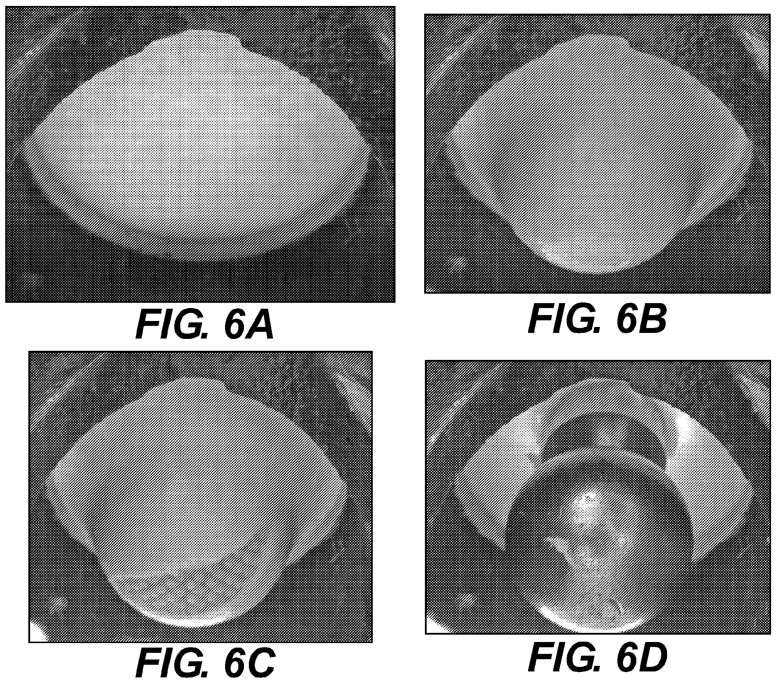

Fig. 6 depicts images of burst strength testing of an exemplary coated polymeric material. Panel A shows the coated material at the beginning of the test. Panel B shows the coated material being stretched by a metal ball. Panel C shows the coated material at the point the coating cracks. Panel D shows the point at which the coated material (including the polypropylene material) breaks completely.

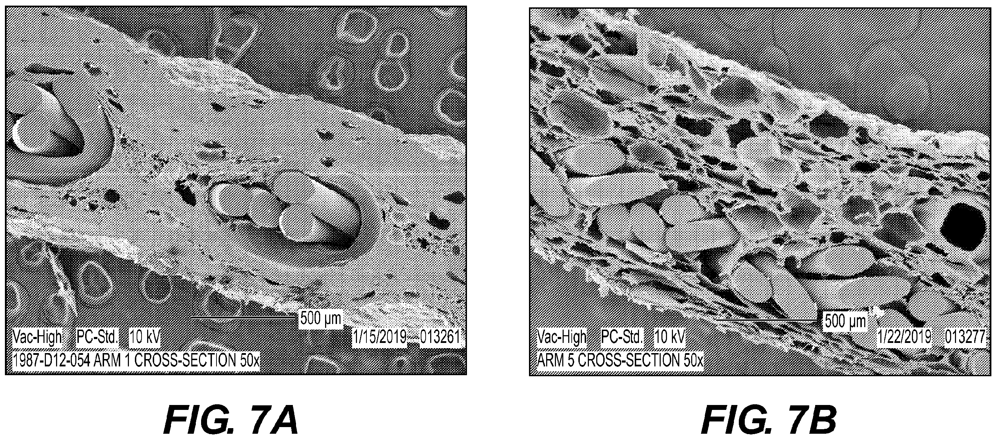

Fig. 7 provides scanning electron microscopy (SEM) images of exemplary coated polymeric materials. The density and porosity of the coating around the polymeric material can be modified by changing the concentrations of the acellular tissue matrix particles, transglutaminase, and at least partially denatured collagen. The coated materials depicted in Fig. 7 include a coating of acellular dermal tissue matrix particles, transglutaminase, and gelatin. The polymeric material is polypropylene. Panel A shows a denser, less porous coating with a high concentration of acellular dermal tissue matrix particles, transglutaminase, and gelatin (5%, 1% and 3%, respectively). Panel B shows a less dense, more porous coating with a lower concentration of acellular dermal tissue matrix particles, transglutaminase, and gelatin (2.5%, 0.5% and 1.5%, respectively).

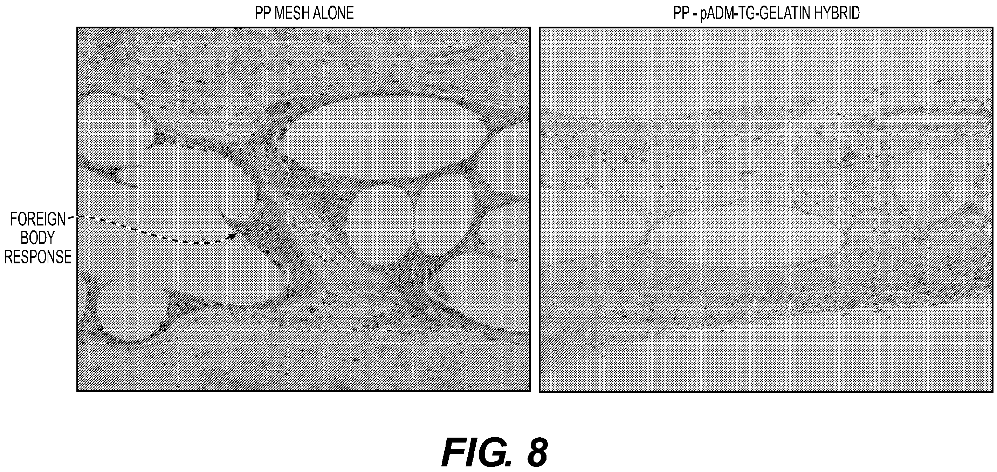

Fig. 8 includes hematoxylin & eosin stained sections of polypropylene materials versus an exemplary coated polymeric material after implantation in a rat. The presence of a foreign body response was evoked by implantation of polypropylene alone but was not present after implantation of the exemplary coated material, in a rat subcutaneous model.

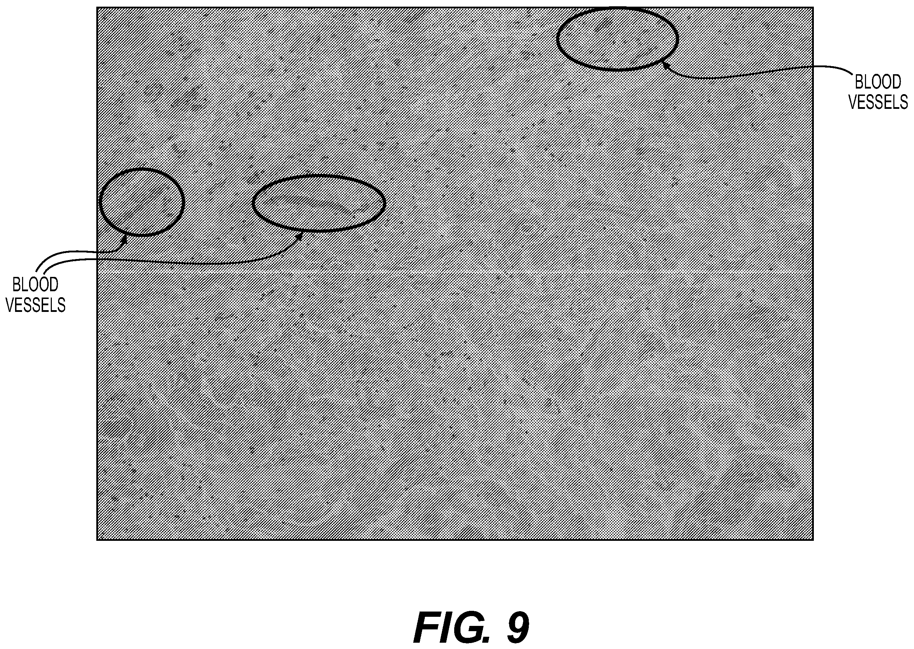

Fig. 9 includes hematoxylin & eosin stained sections of an exemplary coated polymeric material after implantation in a rat. The sections depict cell infiltration, vascularization, and minimal inflammation in a rat subcutaneous model. Blood vessel formations are highlighted. The rat tissue was harvested twelve weeks after implantation.

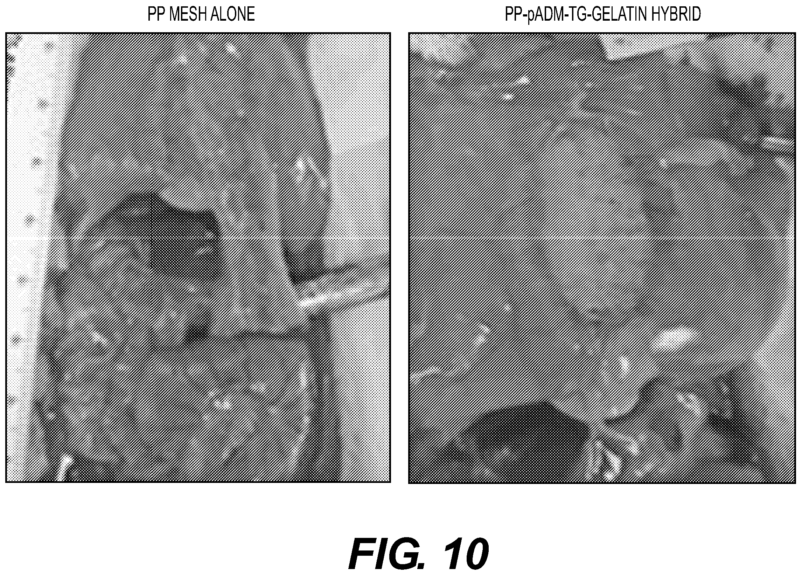

Fig. 10 are gross images of expalnts of a polypropylene implant material versus an exemplary coated polymeric material after 4 weeks implantation in a rat abdominal wall full thickness defect model. The coating prevented visceral adhesion that occurred in the case of uncoated polypropylene material.

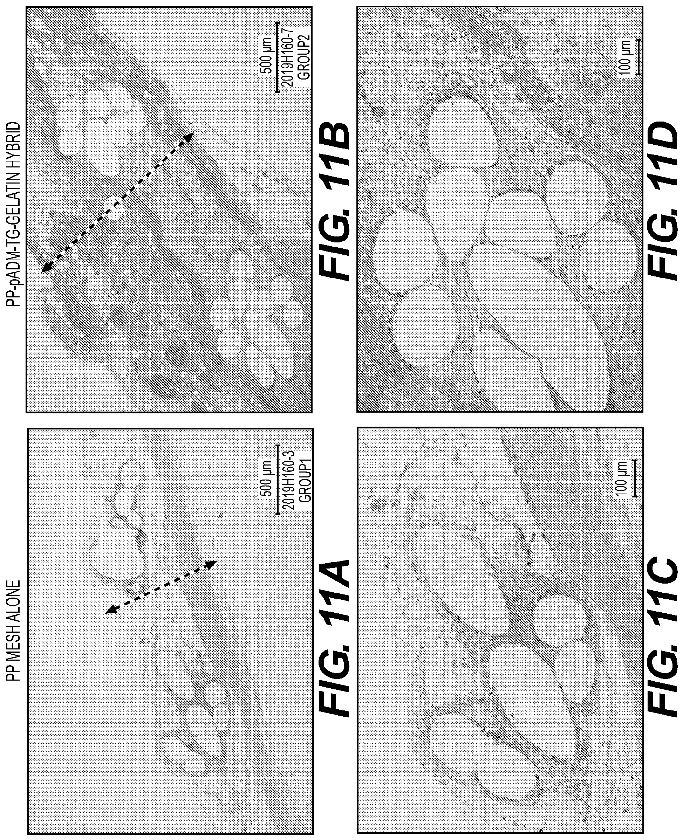

Fig. 11 includes hematoxylin & eosin stained sections of a polypropylene material versus an exemplary coated polymeric material after 4 weeks implantation in a rat abdominal wall full thickness defect model. The exemplary coated polymeric material led to thicker tissue incorporation as compared to uncoated polypropylene mesh (Panels A and C). Inflammation and foreign body response evoked by polypropylene mesh was greatly reduced after implantation of the exemplary coated material (Panels B and D).

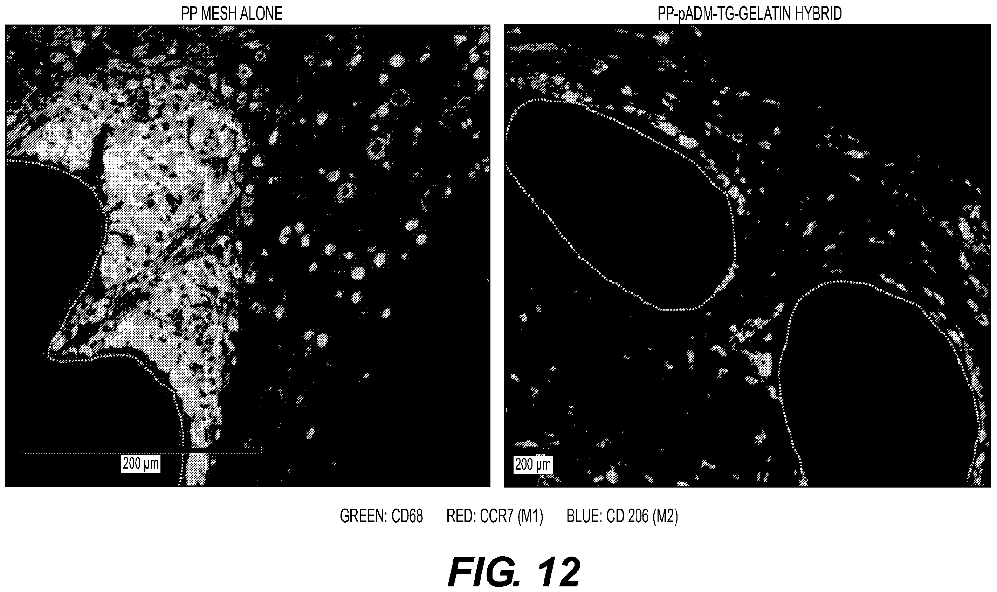

Fig. 12 depicts immunofluorescence stained sections using antibodies against specific macrophage phenotypic markers on a polypropylene material versus an exemplary coated polymeric material after 4 weeks implantation in a rat abdominal wall full thickness defect model. The uncoated polypropylene mesh evoked predominantly a pro-inflammatory M1 macrophage response. The exemplary coated material did not evoke a M1 macrophage response around the polymer material and instead promoted a pro-remodeling M2 macrophage response in the surrounding tissue.

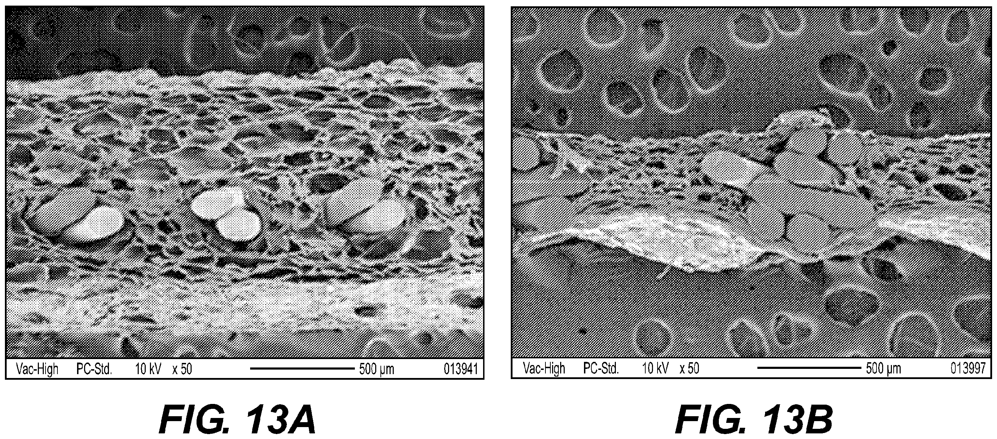

Fig. 13 provides scanning electron microscopy (SEM) images of exemplary coated polymeric material at different coating thicknesses. The coating thickness can be controlled by adjusting the amount of slurry poured around the polymeric material.

The above description and embodiments are exemplary only and should not be construed as limiting the intent and scope of the invention.

## Claims

1. A tissue composition, comprising:
a polymeric material; and
a coating disposed on at least a surface of the polymeric material, the coating comprising:
a group of acellular tissue matrix particles;
transglutaminase; and
an at least partially denatured collagen, wherein the acellular tissue matrix particles are mixed with transglutaminase and at least partially denatured collagen, and the mixture is disposed on at least a surface of the polymeric material.

2. The composition of claim 1, wherein the group of acellular tissue matrix particles comprise acellular dermal tissue matrix particles.

3. The composition of claim 1, wherein the group of acellular tissue matrix particles comprise porcine acellular tissue matrix particles.

4. The composition of claim 1, wherein the group of acellular tissue matrix particles are treated with an enzymatic solution.

5. The composition of claim 4, wherein the enzymatic solution comprises a proteolytic enzyme.

6. The composition of claim 1, wherein the composition is freeze-dried.

7. The composition of claim 1, wherein the coating comprises about 0.1% to 25% of the acellular tissue matrix particles.

8. The composition of claim 1, wherein the coating comprises about 0.5% to 10% of the transglutaminase.

9. The composition of claim 1, wherein the coating comprises about 0.5% to 10% of the at least partially denatured collagen.

10. The composition of claim 1, wherein the polymeric material is a synthetic polymer.

11. The composition of claim 1, wherein the polymeric material is biodegradeable.

12. The composition of claim 1, wherein the polymeric material is polypropylene.

13. The composition of claim 1, wherein the at least partially denatured collagen is a gelatin.

14. The composition of claim 13, wherein the gelatin is a transglutaminase treated gelatin.

15. The composition of claim 6, wherein the freeze-dried composition is stabilized with dehydrothermal treatment.

## Patentansprüche

1. Gewebezusammensetzung, umfassend:
ein Polymermaterial; und
eine Beschichtung, die auf mindestens einer Oberfläche des Polymermaterials angeordnet ist, wobei die Beschichtung umfasst:
eine Gruppe von azellulären Gewebematrixpartikeln;
Transglutaminase; und
ein mindestens teilweise denaturiertes Kollagen, wobei die azellulären Gewebematrixpartikel mit Transglutaminase und mindestens teilweise denaturiertem Kollagen vermischt sind, und die Mischung auf mindestens einer Oberfläche des Polymermaterials angeordnet ist.

2. Zusammensetzung nach Anspruch 1, wobei die Gruppe der azellulären Gewebematrixpartikel azelluläre dermale Gewebematrixpartikel umfasst.

3. Zusammensetzung nach Anspruch 1, wobei die Gruppe der azellulären Gewebematrixpartikel azelluläre Gewebematrixpartikel vom Schwein umfasst.

4. Zusammensetzung nach Anspruch 1, wobei die Gruppe von azellulären Gewebematrixpartikeln mit einer enzymatischen Lösung behandelt wird.

5. Zusammensetzung nach Anspruch 4, wobei die enzymatische Lösung ein proteolytisches Enzym umfasst.

6. Zusammensetzung nach Anspruch 1, wobei die Zusammensetzung gefriergetrocknet ist.

7. Zusammensetzung nach Anspruch 1, wobei die Beschichtung etwa 0,1 % bis 25 % der azellulären Gewebematrixpartikel umfasst.

8. Zusammensetzung nach Anspruch 1, wobei die Beschichtung etwa 0,5 % bis 10 % der Transglutaminase umfasst.

9. Zusammensetzung nach Anspruch 1, wobei die Beschichtung etwa 0,5 % bis 10 % des mindestens teilweise denaturierten Kollagens umfasst.

10. Zusammensetzung nach Anspruch 1, wobei das Polymermaterial ein synthetisches Polymer ist.

11. Zusammensetzung nach Anspruch 1, wobei das Polymermaterial biologisch abbaubar ist.

12. Zusammensetzung nach Anspruch 1, wobei das Polymermaterial Polypropylen ist.

13. Zusammensetzung nach Anspruch 1, wobei das mindestens teilweise denaturierte Kollagen eine Gelatine ist.

14. Zusammensetzung nach Anspruch 13, wobei die Gelatine eine mit Transglutaminase behandelte Gelatine ist.

15. Zusammensetzung nach Anspruch 6, wobei die gefriergetrocknete Zusammensetzung mit einer dehydrothermalen Behandlung stabilisiert wird.

## Revendications

1. Composition tissulaire, comprenant :
un matériau polymère ; et
un revêtement disposé sur au moins une surface du matériau polymère, le revêtement comprenant :
un groupe de particules de matrice tissulaire acellulaire ;
de la transglutaminase ; et
un collagène au moins partiellement dénaturé, dans laquelle les particules de matrice tissulaire acellulaire sont mélangées avec de la transglutaminase et du collagène au moins partiellement dénaturé, et le mélange est disposé sur au moins une surface du matériau polymère.

2. Composition selon la revendication 1, dans laquelle le groupe de particules de matrice tissulaire acellulaire comprend des particules de matrice tissulaire dermique acellulaire.

3. Composition selon la revendication 1, dans laquelle le groupe de particules de matrice tissulaire acellulaire comprend des particules de matrice tissulaire acellulaire porcine.

4. Composition selon la revendication 1, dans laquelle le groupe de particules de matrice tissulaire acellulaire est traité avec une solution enzymatique.

5. Composition selon la revendication 4, dans laquelle la solution enzymatique comprend une enzyme protéolytique.

6. Composition selon la revendication 1, dans laquelle la composition est séchée par lyophilisation.

7. Composition selon la revendication 1, dans laquelle le revêtement comprend environ 0,1 % à 25 % des particules de matrice tissulaire acellulaire.

8. Composition selon la revendication 1, dans laquelle le revêtement comprend environ 0,5 % à 10 % de la transglutaminase.

9. Composition selon la revendication 1, dans laquelle le revêtement comprend environ 0,5 % à 10 % du collagène au moins partiellement dénaturé.

10. Composition selon la revendication 1, dans laquelle le matériau polymère est un polymère synthétique.

11. Composition selon la revendication 1, dans laquelle le matériau polymère est biodégradable.

12. Composition selon la revendication 1, dans laquelle le matériau polymère est du polypropylène.

13. Composition selon la revendication 1, dans laquelle le collagène au moins partiellement dénaturé est une gélatine.

14. Composition selon la revendication 13, dans laquelle la gélatine est une gélatine traitée par transglutaminase.

15. Composition selon la revendication 6, dans laquelle la composition de lyophilisation est stabilisée avec un traitement déhydrothermique.
